# EUROPEAN PATENT SPECIFICATION

(11) **EP 3 717 914 B1**
(45) Date of publication and mention of the grant of the patent: **01.10.2025**
(21) Application number: 18811907.7
(22) Date of filing: 26.11.2018
(51) Int. Cl.: G01N 33/574, G01N 33/497

(54) **DETECTION OF BIOMARKERS**
DETEKTION VON BIOMARKERN
DÉTECTION DE BIOMARQUEURS

(30) Priority: 27.11.2017 GB 201719639
(43) Date of publication of application: 07.10.2020
(73) Proprietor: IP2IPO Innovations Limited, London EC4N 8AF (GB)
(72) Inventor: HANNA, George, London W2 1NY (GB); BOSHIER, Piers, London W2 1NY (GB); BELLUOMO, Ilaria, London W2 1NY (GB)
(74) Representative: Hutter, Anton
(86) International application number: PCT/GB2018/053407
(87) International publication number: WO 2019/102221

(56) References cited:
- EP-A1- 3 009 838
- WO-A1-00/57182
- WO-A1-03/015631
- WO-A1-2010/079490
- WO-A1-2012/045150
- WO-A2-2004/006766
- WO-A2-2004/081527
- US-A- 4 772 559
- US-A1- 2002 039 599
- US-A1- 2005 150 778
- BUSZEWSKI B ET AL: "Identification of volatile organic compounds secreted from cancer tissues and bacterial cultures", JOURNAL OF CHROMATOGRAPHY B: BIOMEDICAL SCIENCES & APPLICATIONS, ELSEVIER, AMSTERDAM, NL, vol. 868, no. 1-2, 1 June 2008 (2008-06-01), pages 88 - 94, XP022698871, ISSN: 1570-0232, [retrieved on 20080503], DOI: 10.1016/J.JCHROMB.2008.04.038
- PATRIK SPANEL ET AL: "Quantification of trace levels of the potential cancer biomarkers formaldehyde, acetaldehyde and propanol in breath by SIFT-MS", JOURNAL OF BREATH RESEARCH, vol. 2, no. 4, 24 July 2008 (2008-07-24), US, pages 046003, XP055553822, ISSN: 1752-7155, DOI: 10.1088/1752-7155/2/4/046003
- SHERAZ R MARKAR ET AL: "Research protocol for a diagnostic study of non-invasive exhaled breath analysis for the prediction of oesophago-gastric cancer", BMJ OPEN, vol. 6, no. 1, 1 January 2016 (2016-01-01), pages e009139, XP055555026, ISSN: 2044-6055, DOI: 10.1136/bmjopen-2015-009139
- D. F. ALTOMARE ET AL: "Exhaled volatile organic compounds identify patients with colorectal cancer : Breath analysis in colorectal cancer diagnosis", BRITISH JOURNAL OF SURGERY., vol. 100, no. 1, 5 December 2012 (2012-12-05), GB, pages 144 - 150, XP055554841, ISSN: 0007-1323, DOI: 10.1002/bjs.8942

## Description

The present invention relates to the detection of biomarkers, and particularly although not exclusively, to methods and the use of compositions and kits for the detection of biological markers for diagnosing various conditions, such as cancer. In particular, the invention relates to the detection of compounds as diagnostic and prognostic markers for detecting cancer, such as oesophago-gastric cancer.

Oesophageal adenocarcinoma is among the most common five cancers and has the fastest rising incidence of any cancer in the Western population. The UK has the highest incidence of oesophageal adenocarcinoma worldwide. Stomach cancer is the third leading cause of cancer death worldwide. Five-year survival for oesophageal and gastric cancer in the UK remains very poor (13% and 18% respectively), among the worst in Europe. The key to improving cancer-survival is earlier diagnosis. However, symptoms are non-specific and commonly-shared with benign diseases. By the time symptoms become cancer-specific, the disease is often at an advanced stage with poor prognosis. Cancer burden and unnecessary investigations of patients with non-specific symptoms result in substantial costs. There is, thus, an urgent need for a non-invasive test for patients with non-specific gastrointestinal symptoms in order to effectively triage patients to have endoscopy and other diagnostic modalities.

Prior research has shown an association between oesophago-gastric cancer and volatile organic compounds (VOCs), and an approach for its diagnosis is exhaled breath testing. Researchers using gas chromatography mass spectrometry (GC-MS) have suggested the existence of a breath volatile organic compounds (VOCs) profile specific to a specific cancer [4]. GC-MS is a good technique for VOC identification, but it is semiquantitative in nature, unless robust calibration curves employed, and therefore limited in its ability of research findings to be reproduced by different research groups. Furthermore, there is a substantial analytical time for each sample, which does not naturally lend itself to high throughput analysis. Direct injection mass spectrometry, such as selected ion flow tube mass spectrometry (SIFT-MS) and proton transfer reaction time of flight mass spectrometry (PTR-ToF-MS) have the advantage of being quantitative and permit real-time analysis [5,6]. WO2004/0067766 discloses the detection of Helicobacter pylori by analysing breath samples of patients after ingestion of urea.

What is required is a reliable non-invasive diagnostic test to identify patients suffering from cancers, such as oesophago-gastric cancer. A diagnostic method to identify those patients with cancer would be of immense benefit to patients and would raise the possibility of early treatment and improved prognosis.

The inventors have developed a non-invasive test for cancer based on the detection of signature compounds, such as volatile organic compounds (VOCs), in exhaled breath. Improved accuracy of this test is achieved by means of administering an oral stimulus foodstuff (e.g. a drink, capsule or solid foodstuff), which transiently induces or "stimulates" cancer and its associated microbiome to produce greater quantities of distinctive signature compounds (e.g. VOCs), and thereby improving test performance and diagnostic accuracy. This will allow patients with non-specific symptoms, yet at a high-risk of oesophago-gastric cancer, to be identified earlier and referred for further investigation and treatment.

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments for use in a method of treatment of the human (or animal) body by therapy (of for diagnosis).

In a first aspect of the invention, there is provided a method for diagnosing a subject suffering from oesophago-gastric junction cancer, gastric cancer, oesophageal cancer, oesophageal squamous-cell carcinoma (ESCC), or oesophageal adenocarcinoma (EAC), or a pre-disposition thereto, or for providing a prognosis of the subject's cancer, the method comprising:
(i) detecting, in a breath sample obtained from a test subject, the concentration of a signature compound resulting from the metabolism, by a cancer-associated microorganism, of at least one substrate in a composition previously administered to the subject, wherein the cancer-associated microorganism is *Streptococcus, Lactobacillus, Veillonella, Prevotella, Neisseria, Haemophilus, L. coleohominis, Lachnospiraceae, Klebsiella, Clostridiales, or Erysipelotrichales,* or any combination thereof, and the at least one substrate is selected from the group consisting of: glucose, sorbitol, lactose, tyrosine, glutamic acid, glycerol, citric acid and acetic acid, or any combination thereof; and
(ii) comparing this concentration with a reference for the concentration of the signature compound in an individual who does not suffer from cancer,

wherein the signature compound is selected from the group consisting of: acetone, acetic acid, butyric acid, pentanoic acid, hexanoic acid, phenol, ethyl phenol, acetaldehyde, or any combination thereof, and
wherein an increase or a decrease in the concentration of the signature compound compared to the reference, suggests that the subject is suffering from cancer, or has a pre-disposition thereto, or provides a negative prognosis of the subject's condition.

In a second aspect of the invention, there is provided the use of a composition in the in *vitro* diagnosis or prognosis of oesophago-gastric junction cancer, gastric cancer, oesophageal cancer, oesophageal squamous-cell carcinoma (ESCC), or oesophageal adenocarcinoma (EAC), according to the method of the first aspect, wherein the composition consists of one or more substrate selected from the group consisting of: glucose, sorbitol, lactose, tyrosine, glutamic acid, glycerol, citric acid and acetic acid, or any combination thereof, and wherein the at least one substrate is metabolised by a cancer-associated microorganism into a signature compound selected from the group consisting of: acetone, acetic acid, butyric acid, pentanoic acid, hexanoic acid, phenol, ethyl phenol, acetaldehyde, or any combination thereof.

In a third aspect, there is provided the use of a kit in the *in vitro* diagnosis of oesophago-gastric junction cancer, gastric cancer, oesophageal cancer, oesophageal squamous-cell carcinoma (ESCC), or oesophageal adenocarcinoma (EAC), or a pre- disposition thereto, or in the prognosis of oesophago-gastric junction cancer, gastric cancer, oesophageal cancer, oesophageal squamous-cell carcinoma (ESCC), or oesophageal adenocarcinoma (EAC), wherein the kit is for performing the method according to the first aspect, the kit comprising:-
(a) a composition consisting of one or more substrate selected from the group consisting of: glucose, sorbitol, lactose, tyrosine, glutamic acid, glycerol, citric acid and acetic acid, or any combination thereof;
(b) means for determining the concentration of a signature compound resulting from the metabolism, by a cancer-associated microorganism, of the at least one substrate, in a breath sample from a test subject, wherein the signature compound is selected from the group consisting of: acetone, acetic acid, butyric acid, pentanoic acid, hexanoic acid, phenol, ethyl phenol, acetaldehyde, or any combination thereof; and
(c) a reference for the concentration of the signature compound in a breath sample from an individual who does not suffer from cancer,
wherein the kit is used to identify an increase or a decrease in the concentration of the signature compound in the breath sample from the test subject, compared to the reference, thereby suggesting that the subject suffers from cancer, or has a pre-disposition thereto, or provides a negative prognosis of the subject's condition.

The following examples are outside the subject matter of the claims and are for information only.

In an example, there is provided a method for diagnosing a subject suffering from cancer, or a pre-disposition thereto, or for providing a prognosis of the subject's condition, the method comprising:
(i) detecting, in a bodily sample from a test subject, the concentration of a signature compound resulting from the metabolism by a cancer-associated microorganism, of at least one substrate in a composition previously administered to the subject; and
(ii) comparing this concentration with a reference for the concentration of the signature compound in an individual who does not suffer from cancer,
wherein an increase or a decrease in the concentration of the signature compound compared to the reference, suggests that the subject is suffering from cancer, or has a pre-disposition thereto, or provides a negative prognosis of the subject's condition.

In an example, there is provided a method for detecting a signature compound in a test subject, the method comprising:
(i) providing the subject with a composition comprising at least one substrate which is suitable for metabolism by cancer-associated microorganism into a signature compound; and
(ii) detecting the concentration of the signature compound in a bodily sample from the subject.

In an example, there is provided a composition comprising at least one substrate which is suitable for metabolism by a cancer-associated microorganism into a signature compound, for use in a method of diagnosis or prognosis, preferably of cancer.

In an example, there is provided a composition comprising at least one substrate which is suitable for metabolism by a cancer-associated microorganism into a signature compound, for use in the method of the above examples.

In an example, there is provided a kit for diagnosing a subject suffering from cancer, or a pre-disposition thereto, or for providing a prognosis of the subject's condition, the kit comprising:-
(a) a composition comprising at least one substrate which is suitable for metabolism by cancer-associated microorganism into a signature compound;
(b) means for determining the concentration of a signature compound in a sample from a test subject; and
(c) a reference for the concentration of the signature compound in a sample from an individual who does not suffer from cancer,
wherein the kit is used to identify an increase or a decrease in the concentration of the signature compound in the bodily sample from the test subject, compared to the reference, thereby suggesting that the subject suffers from cancer, or has a pre-disposition thereto, or provides a negative prognosis of the subject's condition.

Methods of the disclosure may comprise administering or having administered, to the subject, a therapeutic agent or putting the subject on a specialised diet, wherein the therapeutic agent or the specialised diet prevents, reduces or delays progression of cancer.

Thus, in another example, there is provided a method of treating a subject suffering from cancer, said method comprising the steps of:
(i) providing the subject with a composition comprising at least one substrate which is suitable for metabolism by a cancer-associated microorganism into a signature compound;
(ii) analysing the concentration of the signature compound in a bodily sample from a test subject and comparing this concentration with a reference for the concentration of the signature compound in an individual who does not suffer from cancer, wherein an increase or a decrease in the concentration of the signature compound in the bodily sample from the test subject compared to the reference suggests that the subject is suffering from cancer, or has a pre-disposition thereto, or has a negative prognosis; and
(iii) administering or having administered, to the subject, a therapeutic agent or putting the subject on a specialised diet, wherein the therapeutic agent or the specialised diet prevents, reduces or delays progression of cancer.

In another example, there is provided a method for determining the efficacy of treating a subject suffering from cancer with a therapeutic agent or a specialised diet, the method comprising:
(i) providing the subject with a composition comprising at least one substrate which is suitable for metabolism by a cancer-associated microorganism into a signature compound; and
(ii) analysing the concentration of the signature compound in a bodily sample from a test subject, and comparing this concentration with a reference for the concentration of the signature compound in an individual who does not suffer from cancer,
wherein an increase or a decrease in the concentration of the signature compound in the bodily sample from the test subject compared to the reference suggests that the treatment regime with the therapeutic agent or the specialised diet is effective or ineffective.

Advantageously, the benefits for patients with cancer are earlier detection in a population who may have vague or undetectable symptoms. The diagnostic or prognostic methods of the invention can be offered immediately by a medical professional in a similar manner to a routine blood test, thus avoiding the need to "watch-and-wait" to see if a patient's symptoms worsen. Earlier detection significantly improves survival rates. It will be appreciated that "diagnosis" can mean the identification of the nature of an illness or condition, and that "prognosis" can mean predicting the rate of progression or improvement and/or duration of the condition. A prognosis method may be performed subsequent to, and separately from, an initial diagnosis.

Furthermore, the methods could increase the proportion of appropriate referrals from primary care to have endoscopy and could potentially improve referral guidelines. The methods will also provide the opportunity to test younger patients than NICE-age threshold for referral. Patients without cancer will avoid invasive tests and anxiety while awaiting endoscopy. Avoiding unnecessary investigations would free up resources that could be used to save lives. Enhancing the diagnostic pathway will improve patient experience.

Preferably, the methods comprise an initial step of providing the subject with the composition comprising the at least one substrate which is suitable for metabolism by a cancer-associated microorganism into a signature compound. Preferably, the methods of the invention comprise analysing the concentration of the signature compound in the bodily sample from the test subject and comparing this concentration with the reference for the concentration of the signature compound in an individual who does not suffer from cancer.

According to the invention, the cancer-associated microorganism is associated with oesophago-gastric junction cancer, gastric cancer, oesophageal cancer, oesophageal squamous-cell carcinoma (ESCC), or oesophageal adenocarcinoma (EAC). Therefore, the diagnosis is for diagnosing oesophago-gastric junction cancer, gastric cancer, oesophageal cancer, oesophageal squamous-cell carcinoma (ESCC), or oesophageal adenocarcinoma (EAC). Most preferably, the micro-organism is associated with oesophago-gastric cancer, such that this condition can be diagnosed or prognosed.

In an example, the cancer-associated microorganism is associated with pancreatic cancer or colorectal cancer. Accordingly, the diagnosis may be for diagnosing pancreatic cancer or colorectal cancer.

The methods of the invention are useful for monitoring the efficacy of a putative treatment for the relevant cancer. For example, the treatment for resectable oesophago-gastric cancer may comprise neoadjuvant chemotherapy, or chemoradiotherapy followed by surgery and adjuvant chemotherapy. The treatment for very early stage oesophago-gastric cancer may comprise endoscopic resection. The treatment for advanced oesophago-gastric cancer may comprise palliative chemotherapy. It has recently been shown that cancer-associated microbiome enhances metastasis to the liver (Bullman et al., Science, 2017). Hence, the invention described herein may be used to monitor the response of therapy directed towards the cancer-associated microbiome.

If the cancer is pancreatic cancer, then treatment may comprise administration of chemotherapy, chemoradiotherapy with or without surgery. For example, if the cancer is colorectal cancer, then treatment may comprise administration of chemotherapy, chemoradiotherapy with or without surgery, or endoscopic resection.

The composition comprising at least one substrate, which is suitable for metabolism by a cancer-associated microorganism into a signature compound, is ingested by the subject. The composition may be solid or fluid, which may be eaten or swallowed. In an embodiment, the composition may be chewable, which results in release of the substrate and it being taken down into the gut. In an aspect, the composition comprising the at least one substrate may be in the form of a capsule that is designed to degrade at a certain position with the gastrointestinal tract, thereby offering targeted release of the at least one substrate. However, the composition is preferably a liquid (i.e. a drink), which may be swallowed, and which may be referred to as an oral stimulant drink (OSD).

The at least one substrate may be any molecule or compound that can be metabolised by a cancer-associated microorganism, either directly or indirectly, into a signature compound. The at least one substrate may be selected from a group consisting of: tyrosine, acetic acid, ethanol, lactic acid, lactate, glutamic acid, glutamate, glycerol, D-glucose, D-sucrose, D-lactose, D-fructose, D-mannose, D-gulose, D-galactose, D-Xylose, D-arabinose, D-lyxose, D-ribose, L-arabinose, L-rhamnose, L-xylulose, di-, tri-oligo and poly-saccharides, sorbitol, c4, c7 and >c8 monosaccharides, pyruvic acid, ascorbic acid, malic acid, citric acid, succinic acid, fumaric acid, oxalic acid, tannic acid, tartaric acid, sorbitol, mannitol, maltitol, lactitol, erythritol, palmitic acid, stearic acid, oleic acid, linoleic acid, arachidonic acid, triglycerides, glycolipids, any or all of the 20 proteinogenic amino acids, 2-amino butyric acid, ornithine, canavanine, homoarginine, artificial sweeteners (e.g. stevia, aspartame, sucralose), preservatives (e.g. E numbers, nitrate), and small molecule inducers.

The composition may comprise any combination of the aforementioned substrates. Preferably, the composition comprises or consists one or more substrate selected from the group consisting of: glucose, sorbitol, lactose tyrosine, glutamic acid, glycerol, citric acid and acetic acid, or any combination thereof.

Preferably, the composition comprises glucose, preferably at a concentration of between about 7000mg/100mL and 20000mg/100mL, more preferably between 9000mg/100mL and 17000mg/100mL, and most preferably between 11000mg/100mL and 15000mg/100mL.

Preferably, the composition comprises lactose, preferably at a concentration of between about 7000mg/100mL and 20000mg/100mL, more preferably between 9000mg/100mL and 17000mg/100mL, and most preferably between 11000mg/100mL and 15000mg/100mL.

Preferably, the composition comprises sorbitol, preferably at a concentration of between about 1000mg/100mL and 6000mg/100mL, more preferably between 2000mg/100mL and 5000mg/100mL, and most preferably between 3000mg/100mL and 4000mg/100mL.

Preferably, the composition comprises tyrosine, preferably at a concentration of between about 25mg/100mL and 500mg/100mL, more preferably between 50mg/100mL and 400mg/100mL, and most preferably between 100mg/100mL and 300mg/100mL.

Preferably, the composition comprises glutamic acid, preferably at a concentration of between about 500mg/100mL and 5000mg/100mL, more preferably between 1000mg/100mL and 3500mg/100mL, and most preferably between 1500mg/100mL and 2500mg/100mL.

Preferably, the composition comprises glycerol, preferably at a concentration of between about 10000mg/100mL and 30000mg/100mL, more preferably between 14000mg/100mL and 25000mg/100mL, and most preferably between 17000mg/100mL and 22000mg/100mL.

Preferably, the composition comprises citric acid, preferably at a concentration of between about 500mg/100mL and 3000mg/100mL, more preferably between 1000mg/100mL and 2000mg/100mL, and most preferably between 1200mg/100mL and 1700mg/100mL.

Preferably, the composition comprises acetic acid, preferably at a concentration of between about 200mg/100mL and 1500mg/100mL, more preferably between 400mg/100mL and 1000mg/100mL, and most preferably between 600mg/100mL and 800mg/100mL.

Preferably, the composition comprises or consists of at least two, three or four of the substrates selected from the group consisting of tyrosine, glutamic acid, glucose, sorbitol, lactose, glycerol, citric acid and acetic acid. Preferably, the composition comprises or consists of at least five, six, seven or eight of the substrates selected from the group consisting of tyrosine, glutamic acid, glucose, sorbitol, lactose, glycerol, citric acid and acetic acid.

The inventors believe that glucose, lactose and sorbitol are especially useful as substrate in the composition for metabolism by a cancer-associated microorganism into the signature compound. Most preferably, therefore, the composition comprises at least one substrate selected from glucose, lactose and sorbitol. It will be appreciated, however, that tyrosine, glutamic acid, glycerol, citric acid and/or acetic acid may also be included in the composition in any of the above concentrations.

The composition may be an existing composition, foodstuff or drink, which comprises any one of the aforementioned constituents.

The cancer-associated microorganism may be a bacterium. It will be appreciated that the microorganisms and bacteria present in the gut form the so-called "microbiome". Therefore, the cancer-associated microorganism that metabolises the at least one substrate into a signature compound, which is detected and/or analysed in the methods of the invention to diagnose cancer, preferably form part of the microbiome.

The cancer-associated microorganism according to the invention is *Streptococcus, Lactobacillus, Veillonella, Prevotella, Neisseria, Haemophilus, L. coleohominis, Lachnospiraceae, Klebsiella, Clostridiales, Erysipelotrichales,* or any combination thereof.

The cancer-associated microorganism may be *S. pyogenes, Klebsiella pneumoniae, Lactobacillus acidophilus,* or any combination thereof.

The cancer-associated microorganism described herein may be *E. coli, P. mirabili, B. cepacia, S. pyogenes, Streptococcus salivarius, Actinomyces naeslundii, Lactobacillus fermentum, Streptococcus anginosus, Clostridium bifermentans, Clostridium perfringens, Clostridium septicum, Clostridium sporogenes, Clostridium tertium, Eubacterium lentum, Eubacterium sp., Fusobacterium simiae, Fusobacterium necrophorum, Lactobacillus acidophilus, Peptococcus niger, Peptostreptococcus anaerobius, Peptostreptococcus asaccharolyticus, Peptostreptococcus prevotii, P. aeruginosa, S. aureus, P. mirabilis, E. faecalis, S. pneumoniae, N. meningitides, Acinetobacter baumannii, Bacteroides capillosus, Bacteroides fragilis, Bacteroides pyogenes, Clostridium difficile, Clostridium ramosum, Enterobacter cloacae, Klebsiella pneumoniae, Nocardia sp., Propionibacterium acnes, Propionibacterium propionicum,* or any combination thereof. Preferably, the cancer-associated microorganism is *E. coli, L. fermentum, S. salivarius, S. anginosus* or *K. pneumoniae.*

The subject may be any animal of veterinary interest, for instance, a cat, dog, horse etc.

However, it is preferred that the subject is a mammal, such as a human, either male or female.

Preferably, a sample is taken from the subject, and the signature compound in the bodily sample is then detected. In some embodiments, the concentration of the signature compound is measured.

A signature compound may be any compound that can indicate or correlate with the presence of a microorganism. The signature compounds, which are detected, may be volatile organic compounds (VOCs), which lead to a fermentation profile, and they may be detected in the bodily sample by a variety of techniques. In one embodiment, these compounds may be detected within a liquid or semi-solid sample in which they are dissolved. In a preferred embodiment, however, the compounds are detected from gases or vapours. For example, as the signature compounds are VOCs, they may emanate from, or form part of, the sample, and may thus be detected in gaseous or vapour form.

As described herein, the volatile organic compound (VOC) is selected from a group consisting of: butyric acid, gamma amino butyric acid, caproic acid, hydrogen sulphide, pentanol, propanoic acid, acetic acid, 1,2-propanediol, ethanol, and 3-hydroxypropionic acid, or any combination thereof.

The VOCs may be aldehydes, fatty acids, alcohols, or any combination thereof.

The VOCs may be a C₁-C₃ aldehyde, a C₁-C₃ alcohol, a C₂-C₁₀ alkane wherein a first carbon atom is substituted with the =O group and a second carbon atom is substituted with an -OH group, a C₁-C₂₀ alkane, a C₄-C₁₀ alcohol, a C₁-C₆ carboxylic acid, a C₄-C₂₀ aldehyde, a C₂ aldehyde, a C₅ aldehyde, a C₈ aldehyde, a C₉ aldehyde, a C₁₀ aldehyde, a C₁₁ aldehyde, an analogue or derivative of any aforementioned species, or any combination thereof. The VOCs may be propanal, nonanal, decanal, formaldehyde, methanol, pentane, isopropyl alcohol, n-hexane, 1-butanol, acetoin, propanoic acid, undecanal, tetradecane, or any combination thereof. According to the invention, the VOCs are acetone, acetic acid, butyric acid, pentanoic acid, hexanoic acid, phenol, ethyl phenol, acetaldehyde, or any combination thereof. In yet another example, the VOCs may be hexanoic acid, pentanoic acid, acetic acid, 2 ethyl phenol, or any combination thereof.

In one example, a composition comprising acetic acid and/or ethanol (i.e. the substrate which is metabolised by the cancer-associated microorganism) may be provided to a subject, in order to increase the concentration of the signature compound, butyric acid and/or caproic acid. The concentrations of these signature compounds may then be analysed in order to indicate the presence of *Clostridium spp.* and hence provide a diagnosis, indicate a pre-disposition thereto, or provide a prognosis of, for instance, oesophageal squamous-cell carcinoma. Evidence for the association is shown in Example 3 below. Hence, preferably the method is used to provide a diagnosis, indicate a pre-disposition thereto, or provide a prognosis of, oesophageal squamous-cell carcinoma, wherein the composition comprises a substrate selected from acetic acid and/or ethanol, which is preferably metabolised to a signature compound selected from butyric acid and/or caproic acid, which is preferably analysed to indicate the presence of *Clostridium spp.*

In another example, a composition comprising lactic acid (i.e. the substrate) may be provided to a subject, in order to increase the concentration of the signature compound, acetic acid, 1,2-propanediol, and/or ethanol. The concentration of these signature compounds may then be analysed in order to indicate the presence of *Lactobacillus spp.* and hence provide a diagnosis, indicate a pre-disposition thereto, or provide a prognosis of, for instance, gastric cancer. Evidence for the association is shown in Example 3 below. Accordingly, preferably the method is used to provide a diagnosis, indicate a pre-disposition thereto, or provide a prognosis of, gastric cancer, wherein the composition comprises a substrate which is lactic acid, which is preferably metabolised to a signature compound selected from acetic acid, 1,2-propanediol, and/or ethanol, which is preferably analysed to indicate the presence of *Lactobacillus spp.*

In yet another example, a composition comprising glutamate (i.e. the substrate) may be provided to a subject, in order to increase the concentration of the signature compound, gamma amino butyric acid. The concentration of this signature compound may then be analysed in order to indicate the presence of *Lactococcus spp., Clostridium spp.,* and others, and hence provide a diagnosis, indicate a pre-disposition thereto, or provide a prognosis of oesophago-gastric cancer. Evidence for the association is shown in Example 3 below. Thus, preferably the method is used to provide a diagnosis, indicate a pre-disposition thereto, or provide a prognosis of, oesophago-gastric cancer, wherein the composition comprises a substrate which is glutamate, which is preferably metabolised to a signature compound which is gamma amino butyric acid, which is preferably analysed to indicate the presence of *Lactococcus spp.* or *Clostridium spp.*

In still another example, a composition comprising glycerol (i.e. the substrate) may be provided to a subject, in order to increase the concentration of the signature compound, 3-hydroxypropionic acid. The concentration of this signature compound may then be analysed in order to indicate the presence of *Klebsiella spp.* and hence provide a diagnosis, indicate a pre-disposition thereto, or provide a prognosis of gastric cancer. Evidence for the association is shown in Example 3 below. Therefore, preferably the method is used to provide a diagnosis, indicate a pre-disposition thereto, or provide a prognosis of, gastric cancer, wherein the composition comprises a substrate which is glycerol, which is preferably metabolised to a signature compound which is 3-hydroxypropionic acid, which is preferably analysed to indicate the presence of *Klebsiella spp.*

The kit of the disclosure may comprise sample extraction means for obtaining the sample from the test subject. The sample extraction means may comprise a needle or syringe or the like. The kit may comprise a sample collection container for receiving the extracted sample, which may be liquid, gaseous or semi-solid. The kit may further comprise instructions for use.

As described herein, the sample is any bodily sample into which the signature compound is present or secreted. Preferably, the detection or diagnostic method is therefore performed *in vitro.* For example, the sample may comprise urine, faeces, hair, sweat, saliva, blood, or tears. In one embodiment, the sample may be assayed for the signature compound's levels immediately. Alternatively, the sample may be stored at low temperatures, for example in a fridge or even frozen before the concentration of signature compound is determined. Measurement of the signature compound in the bodily sample may be made on the whole sample or a processed sample, for instance whole blood or processed blood.

In an example, the sample may be a urine sample. It is preferred that the concentration of the signature compound in the bodily sample is measured *in vitro* from a urine sample taken from the subject. The compound may be detected from gases or vapours emanating from the urine sample. It will be appreciated that detection of the compound in the gas phase emitted from urine is preferred.

It will also be appreciated that "fresh" bodily samples may be analysed immediately after they have been taken from a subject. Alternatively, the samples may be frozen and stored. The sample may then be de-frosted and analysed at a later date.

According to the invention, the bodily sample is a breath sample from the test subject. The sample may be collected by the subject performing exhalation through the mouth, preferably after nasal inhalation. Preferably, the sample comprises the subject's alveolar air. Preferably, the alveolar air is collected over dead space air by capturing end-expiratory breath. VOCs from breath bags are then preferably pre-concentrated onto thermal desorption tubes by transferring breath across the tubes.

The difference in concentration of signature compound, which would indicate cancer in the subject or a predisposition thereto, may be an increase or a decrease compared to the reference. It will be appreciated that the concentration of signature compound in patients suffering from a disease is highly dependent on a number of factors, for example how far the disease has progressed, and the age and gender of the subject. It will also be appreciated that the reference concentration of signature compound in individuals who do not suffer from the disease may fluctuate to some degree, but that on average over a given period of time, the concentration tends to be substantially constant. In addition, it should be appreciated that the concentration of signature compound in one group of individuals who suffer from a disease may be different to the concentration of that compound in another group of individuals who do not suffer from the disease. However, it is possible to determine the average concentration of signature compound in individuals who do not suffer from the cancer, and this is referred to as the reference or 'normal' concentration of signature compound. The normal concentration corresponds to the reference values discussed above.

In one embodiment, the methods of the invention preferably comprise determining the ratio of chemicals within the breath (i.e. use other components within it as a reference), and then compare these markers to the disease to show if they are elevated or reduced. The signature compound is preferably a volatile organic compound (VOC), which provides a profile, and it may be detected in or from the bodily sample by a variety of techniques. Thus, these compounds may be detected using a gas analyser. Examples of suitable detector for detecting the signature compound preferably includes an electrochemical sensor, a semiconducting metal oxide sensor, a quartz crystal microbalance sensor, an optical dye sensor, a fluorescence sensor, a conducting polymer sensor, a composite polymer sensor, or optical spectrometry.

The inventors have demonstrated that the signature compounds can be reliably detected using gas chromatography, mass spectrometry, GCMS or TOF. Dedicated sensors could be used for the detection step.

The reference values may be obtained by assaying a statistically significant number of control samples (i.e. samples from subjects who do not suffer from the disease). Accordingly, the reference (ii) according to the kit of the disclosure may be a control sample (for assaying).

The apparatus preferably comprises a positive control (most preferably provided in a container), which corresponds to the signature compound(s). The apparatus preferably comprises a negative control (preferably provided in a container). In an aspect, the kit may comprise the reference, a positive control and a negative control. The kit may also comprise further controls, as necessary, such as "spike-in" controls to provide a reference for concentration, and further positive controls for each of the signature compounds, or an analogue or derivative thereof.

Accordingly, the inventors have realised that the difference in concentrations of the signature compound between the reference normal (i.e. control) and increased/decreased levels, can be used as a physiological marker, suggestive of the presence of a disease in the test subject. It will be appreciated that if a subject has an increased/decrease concentration of one or more signature compounds which is considerably higher/lower than the 'normal' concentration of that compound in the reference, control value, then they would be at a higher risk of having the disease, or a condition that was more advanced, than if the concentration of that compound was only marginally higher/lower than the 'normal' concentration.

The skilled technician will appreciate how to measure the concentrations of the signature compound in a statistically significant number of control individuals, and the concentration of compound in the test subject, and then use these respective figures to determine whether the test subject has a statistically significant increase/decrease in the compound's concentration, and therefore infer whether that subject is suffering from the disease for which the subject has been screened.

Reference will now be made, by way of example, to the accompanying Figure, in which:-
**Figure 1** shows an example of an apparatus and a method used for concentrating VOCs from steel breath bags onto thermal desorption tubes;
**Figure 2** shows VOC production by overall species in patient cancer tissues compared to non-cancer control;
**Figure 3** shows high throughput in vitro culture stimulation and VOC sampling protocol; and
**Figure 4** shows examples of elevated VOCs in the headspace of **(A)** *Escherichia coli* in glucose media, **(B)** *Klebsiella pneumonia* in glucose media, **(C)** *Streptococcus salivarius* in glycerol media, and **(D)** *Lactobacillus fermentum* in glycerol media.

Active biotransformation (AB) compared to controls comprising bacterial cultures that were stimulant-free (SFC) and stimulant compositions that were free of bacterial cultures (CFC). Spike A and B contained 3-ethyl phenol and hexanoic acid as internal compound standards as quality measures. Samples with -P denotes a vacuum pump was used to sample the headspace of the biotransformation vessel into thermal desorption tubes. Data is derived from VOC analysis by GC-MS.

### Examples

### Example 1 - Microbial VOCs produced in response to particular stimulants found in food

Table 1 shows VOCs that can be detected to indicate the presence of specific bacteria, and the studies showing this association.

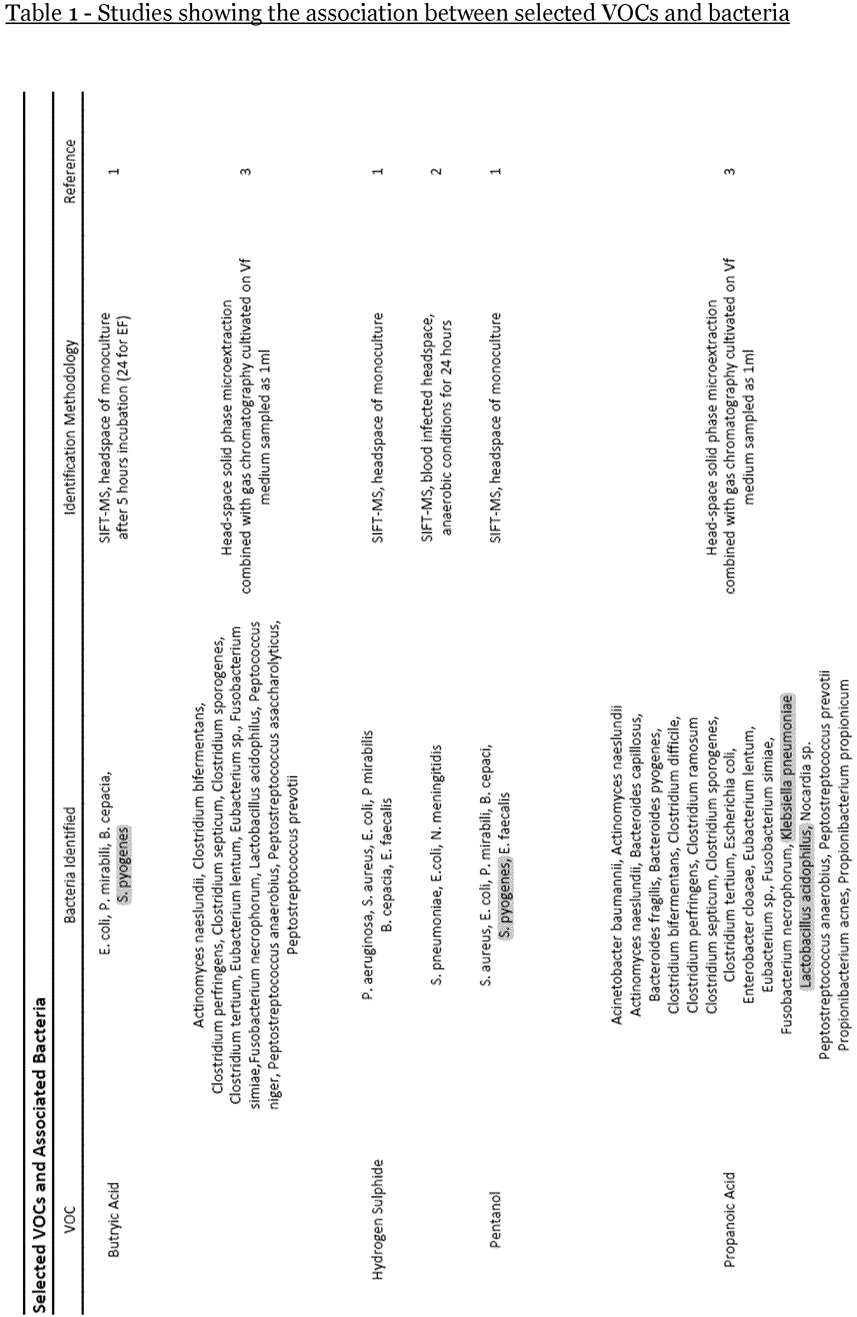

The following provides examples of bacteria whose presence can be indicated by the detection of signature compounds, and examples of the substrates that can be fed to the bacteria to increase the concentration of the signature compounds.

*Clostridium spp.* can be detected by initially feeding the bacteria with substrate compounds, acetic acid, which are metabolised into signature compounds which are detectable. Excess acetic acid produces butyric acid. The signature compound can be measured to thereby detect the presence of *Clostridium spp.*

*Lactobacillus spp.* can be similarly detected by feeding it first with the substrate, lactic acid, which is converted into acetic acid, 1,2-propanediol, and ethanol. These signature compounds can be measured to detect the presence of *Lactobacillus* spp. (not part of the invention).

*Lactobacillus, Clostridia* and other bacteria can be detected by feeding with glutamate.

Glutamate is converted to gamma amino butyric acid. This signature compound can be measured to detect the presence of *Lactococcus spp., Clostridium spp.,* and others (not part of the invention).

*Klebsiella spp.* can be detected by feeding with glycerol. Glycerol is metabolised to 3-hydroxypropionic acid, and this signature compound can be measured to detect the presence of *Klebsiella* spp. (not part of the invention).

### Example 2 - An Augmented Microbiome-mediated Breath Test for the Earlier Diagnosis of Oesopha-gastric Cancer (AMBEC)

The inventors have developed a non-invasive test for oesophago-gastric adenocarcinoma (specificity 81%/sensitivity 80%) based on the detection of volatile organic compounds (VOCs) in exhaled breath. The inventors have improved the accuracy of this test by means of an oral drink which induces the cancer-associated microbiome to produce greater quantities of the distinctive VOCs and thereby allow patients with non-specific symptoms, yet at a high-risk of oesophago-gastric cancer, to be referred faster and earlier for treatment. The oral drink (oral stimulant drink - OSD) selectively 'feeds' the cancer-associated microbiome with substances that it will preferentially metabolise to generate quantifiably higher levels of distinctive VOCs. Briefly, the patient is fasted for at least 4 hours and then, while at rest for 20 minutes, breathes into a bag or using breath collection device (such as ReCIVA - see below) that concentrate the volatile compounds into a thermal desorption tube. Breath samples will be analysed.

The test of the invention could be offered immediately by a medical professional in a similar manner to a routine blood test, thus avoiding the need to "watch-and-wait" to see if a patient's symptoms worsen.

The test is intended to be performed by a medical professional, who would then send breath samples to a laboratory for analysis. A positive result would warrant immediate referral for endoscopy. A negative result would permit the medical professional to reassure the patient and offer retesting if symptoms persist.

One example of the invention is referred to as AMBEC (an Augmented Microbiome-mediated Breath Test for the Earlier Diagnosis of Oesophago-gastric Cancer). The target population for testing with AMBEC is patients with upper gastrointestinal symptoms attending GP practices. AMBEC is a highly patient-friendly non-invasive test that will enable both earlier and faster diagnosis, and will substantially mitigate rising pressures on central diagnostic endoscopy.

Patients are given an oral drink (oral stimulant drink - OSD) to stimulate VOC production by the oesophago-gastric cancer-associated microbiome. The OSD is administered and a breath test is undertaken at 30 minute-intervals for 2 hours.

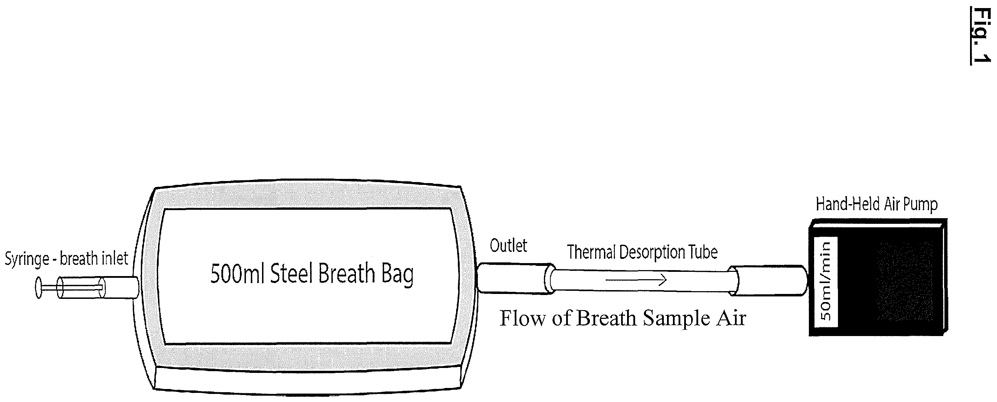

Breath is collected by a low-cost device and analysed in regional laboratories using automated standard mass-spectrometry equipment, such as the apparatus shown in Figure 1.

Referring to Figure 1, there is shown an ReCIVA apparatus used for the breath sampling in accordance with the invention. The ReCIVA apparatus is a reproducible system that allows direct breath collected into the thermal desorption tubes, which is the system to be used in future multi-centre studies.

Breath was collected using 500ml inert aluminium bags that were washed through with synthetic air prior to sampling. Patients were asked to perform deep nasal inhalation followed by complete exhalation through the mouth into secure GastroCHECK steel breath bag. Alveolar air was preferentially collected over dead space air by capturing end-expiratory breath. VOCs from breath bags were then pre-concentrated (see Figure 1) onto thermal desorption tubes by transferring 250ml of breath at 50ml/sec across the tubes with 10mm diameter tubing and hand-held air pumps (210-1002MTX, SKC ltd., Dorset, UK).

Patients are fasted for a minimum of four hours prior to breath sample collection. All breath samples are collected prior to endoscopy or surgery.

Exhaled breath analysis can be performed using GC-MS as the standard identification technique, and PTR-TOF-MS as the quantitative technique with a Time-of-Flight analyser to guarantee cutting-edge performance in terms of mass and time resolution.

### Example 3 - Studies showing the association between cancers and bacteria (E. coli is cited for comparison reasons only)

The studies shown in Table 2 indicate bacteria that have been shown to be associated with particular cancer types. Hence, enabling the diagnosis of these cancers by the detection of these bacteria, for example in a patient's microbiome.

**Table 2 - Bacteria associated with cancer**

| **Author and year of Publication** | **Analytical Platform** | **Cancer site** | **Cancer type** | **Cancer (N)** | **Healthy (N)** | **Bacteria** |
|---|---|---|---|---|---|---|
| Johan Dicksved Microbiology 2009, DOI: 10.1099/jmm.o.o 07302-0. | 16sRNA | Gastric | GC | 6 | 15 | *Streptococcus* |
| | | | | | | *Lactobacillus* |
| | | | | | | *Veillonella* |
| | | | | | | *Prevotella* |
| | | | | | | *Neisseria* |
| | | | | | | *Haemophilus* |
| Francisco Scientific Reports 2014, DOI: 10.1038/srep042 02. | 16sRNA | Gastric | GC | 5 | 5 | *Streptococcus* |
| | | | | | | *Lactobacillus* |
| | | | | | | *Veillonella* |
| | | | | | | *L. coleohomini* |
| | | | | | | *Lachnospiraceae* |
| Chang Soo Eun Helicobacter 2014, DOI: 10.1111/hel.12145. | 16sRNA | Gastric | GC | 11 | 10 | *Streptococcus* |
| | | | | | | *Lactobacillus* |
| | | | | | | *Veillonella* |
| | | | | | | *Prevotella* |
| Yalda Scientific World Journal Volume 2014, DOI: 1 | 16sRNA | Gastric | GC | 8 | 185 | *Streptococci* |
| | | | | | | *Lactobacilli* |
| | | | | | | *Neisseria* |
| | | | | | | *Klebsiella* |
| 421. | | | | | | |
| Nasrollahzadeh Scientific Reports 2015, DOI: 10.1038/srep08 820. | 16sRNA | Esopha gus | ESCC | 37 | 17 | *Clostridiales* |
| | | | | | | *Erysipelotrichales* |

| | | | | | | |
|---|---|---|---|---|---|---|
| Example 3 - Development of Augmented Microbiome-mediated Breath Test for the Earlier Diagnosis of Oesophago-gastric Cancer (AMBEC) (E.coli is cited for comparison reasons only) | | | | | | |

### (i) Production of an oral stimulant drink (OSD)

The aim was to develop an enhanced OSD formulation that enables the inventors to fully optimise the dose-response, reproducibility and robustness of the new triage test for clinical introduction. The production of the OSD was based on: (i) the dataset of gastric microbiomal bacteria most commonly associated with cancer tissue, (ii) an extensive bioinformatics review of the enzymatic pathway regulation and biochemical flux in key bacterial species, (iii) the scientific literature describing the conversion of particular primary metabolites to specific VOCs, and (iv) ethical, safety and acceptability considerations of OSD components, such as normal dietary presence, recommended daily allowance (RDA) and palatability.

Sugars, organic acids and amino acids were identified as priority compounds.

Accordingly, specific stimuli were selected for the initial OSD. Several fatty acid stimuli were discounted due to insolubility within the aqueous OSD formulation. A suitable commercial kitchen was identified for OSD manufacture. All OSD components and consumables were sourced as either "food" or "medical" grade to ensure no possibility of contamination and that the drink is fit for human consumption.

*Results:* Table 3 summarises the composition of one embodiment of the OSD.

**Table 3 - OSD composition**

| | **Stimulant Mix** | | **RDI** |
|---|---|---|---|
| | mg/mL_{water} | mg/kg_{bodyweight}* | mg/kg_{bodyweight}/d |
| *Tyrosine* | 1.75 | 2.5 | 25 |
| *Glutamic Acid* | 21.0 | 30 | 30 |
| *Glucose* | 130.2 | 186 | 1857 ** |
| *Sorbitol* | 35.0 | 50 | |
| *Lactose* | 130.2 | 186 | |
| *Glycerol* | 193.2*** | 276 | |
| *Citric Acid* | 14 | 20 | |
| *Acetic Acid* | 7 | 10 | |

| | | | |
|---|---|---|---|
| The OSD was prepared under ISO9001 principles of Quality Management with full traceability of batch records. The feasibility results from the OSD provide the proposed further work with an excellent basis for the critical intervention necessary to elicit an augmented response. RDI - recommended daily intake * Total bodyweight defined here as 70 kg. ** Estimated for a 70 kg healthy adult. *** ~ 29% lower than glycerol content in Covonia cough syrup. | | | |

In one embodiment, the OSD is in the form of a capsule that is designed to degrade at a certain position with the gastrointestinal tract, thereby offering targeted release of the at least one substrate. In another embodiment, the OSD is a liquid drink. Glucose, lactose and sorbitol are believed to be most important for augmenting the microbiome to produce the signature VOCs.

### (ii) VOC production by microbiome associated with patient cancer types

The aim was to identify dominant microbiome (bacterial species) associated with oesophago-gastric cancer. Dominant microbiomes associated with oesophago-gastric cancer were identified from: (i) a literature search, (ii) 16S analysis of cancer and normal tissue samples, and (iii) microbiome cultures from oesophageal and gastric cancer and non-cancer tissues obtained from oesophagio-gastric cancer and control patients.

### (A) 16S analysis of cancer and normal tissue samples

*Methods:* 16S RNA sequencing analysis was undertaken upon gastric and oesophago-gastric tissue samples obtained during surgery. Samples were subjected to metataxonomic analysis on the Illumina MiSeq platform, with the V3/V4 region of OG cancer microbiomes being targeted in a high-multiplexing approach, thus leading to a high coverage of the microbial diversity. Taxonomic-dependent analysis of reads from amplicon sequencing was performed using Mothur software. Comparison of dominant bacterial phyla within cancer and non-cancer samples using univariate statistical analysis was performed. Supervised and unsupervised statistical modelling was performed with incorporation of clinical metadata.

*Results:* The inventors have identified the presence of a number of bacteria associated with cancer, as shown in Table 4.

**Table 4 - Elevated VOC levels in active biotrans verses controls**

| | **Glycerol media** | **Glucose media** |
|---|---|---|
| ***E. coli*** (NCIMB 9552) | Acetate, propionoic acid, butanoic acid | Hexanal, butanoic acid, pentanoic acid, hexanoic acid |
| ***L. fermentum*** (NCIMB 11840) | Acetaldehyde, Heptanal, ethyl phenol | Acetone |
| ***S*. *Salivarius*** (NCIMB 701779) | Acetate, pentanoic acid, octanal | Insufficient growth |
| ***S.Anginosus*** (NCIMB 702496) | Acetone, Butnaoic acid, Hexanal, Ethyl phenol, Nonanal | Butanoic acid, Hexanal |
| ***K. pneumoniae*** (NCIMB 13281) | Hexanoic acid | Butanoic acid, pentanoic acid |

| | | |
|---|---|---|
| (Stimuli cocktail composition, all at 0.1M concentration: tyrosine, glutamic acid, glucose, lactose, sorbitol, glycerol, ethanol, xylose, phenylalanine) | | |

As shown in Table 4, higher abundance of *Firmicutes* (*Lactobacillus fermentum, Streptococcus salivarius, Streptococcus anginosus, Klebsiella pneumoniae, Escherichia coli*) was found in oesophago-gastric cancer tissue compared to control samples. The identification of dominant oesophago-gastric cancer-associated microbiomes provides target microbiomes to be stimulated by OSD in order to elicit an optimal augmented response.
*(B) Microbiome culture from patient cancer and normal tissue samples* The aim was to illustrate a difference in VOCs originate from bacteria associated with either cancer or normal tissues obtained from patients in order to provide support for the overall hypothesis that the gastric microbiome can produce markers of cancer presence.

*Methods:* Frozen samples of cancer tissue and non-cancer control tissue in glycerol-freeze media were used for Sequencing (16S/Shotgun) and headspace analysis. Tissue samples were defrosted and re-suspended in 100 µL PBS (sterile pH 5) and vortexed vigorously for 60 seconds. 100 µL of supernatant fluid was then spread on pre-prepared FAA (Fastidious Anaerobe Agar + 7% Horse Blood) medium on petri plates and incubated in anaerobic ES-Gas pouches at 37°C for 24 hours. The following day, the plates were removed from the incubator and treated in two ways: (A) Predominant species: Individual bacterial colonies most frequently occurring on each FAA plate were picked and re-suspended in 100 µL PBS in vials for sequencing and headspace analysis; and (B) Overall species composition: 1.5ml PBS was used to re-suspended all bacteria from individual plates. The resuspension was then pipetted into an eppendorf tube and micro-centrifuged at 14800 for 5mins. Supernatant fluid was removed and the solid pellet re-suspended in 100 µL PBS and split into vials for sequencing and headspace analysis via solid phase micro-extraction (SPME- GC-MS) using a carboxen/polydimethysiloxane SPME fibre. SPME extraction was performed at 60°C with intermittent agitation at 500rpm. Volatiles were collected in the absence of airflow, after 48 hours of incubation followed by direct release into a heated gas chromatography injector.

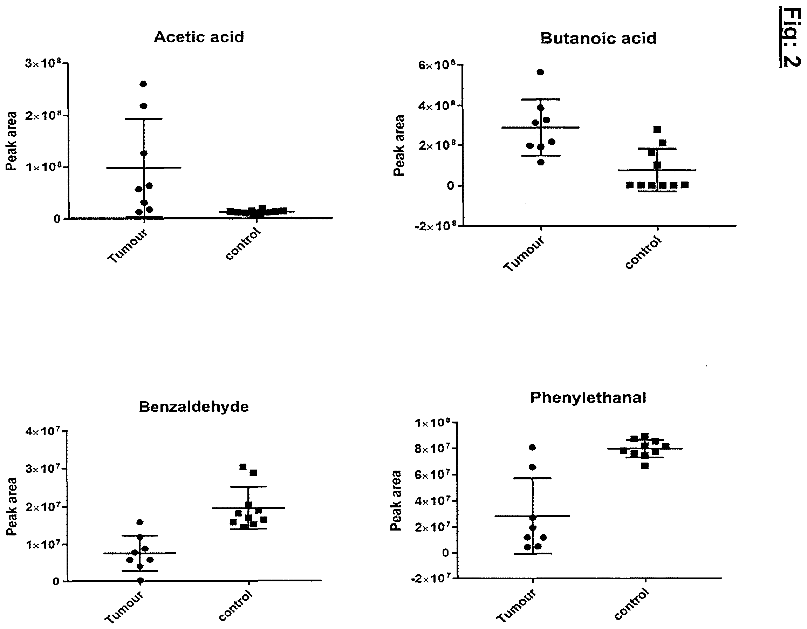

*Results:* Differing abundances of volatile aldehydes and fatty acids were detected in the headspace from predominant and total culturable bacterial species associated with cancer and non-cancer samples. For the predominant bacteria (A), VOCs including benzaldehyde and methyl phenol were significantly higher in samples from cancer tissue versus normal tissue controls. As for the overall bacteria present in the tissue samples (B), acetic and butanoic acid were significantly higher in cancer tissue versus normal controls (see Figure 2).

### (iii) In vitro VOC production by different microbiomes in response to OSD

### A) Standard bacterial culture and VOC production in response to RDA of OSD components

The aim was to examine the feasibility to culture relevant microbiomes and analyse their VOC produced in response to stimuli used in the OSD, at human recommended daily allowance concentrations.

*Methods:* Using the above preliminary clinical data provided by VODCA, and literature references describing microbiome-associations with gastric cancer, suitable strains were obtained from culture collections such as NCIMB (Aberdeen) and ATCC (USA). See Table 4. All *in-vitro* culture work was performed under conditions as closely simulating the natural gastric environment as possible (e.g. anaerobiosis, pH5.5) in Cat1 or Cat2 laboratories as appropriate, according to UK microbiological regulatory guidelines using well-established Ingenza protocols. All associated quality control testing was performed throughout. Several study parameters were optimised during the work to enhance cell growth, culture sampling and VOC analysis.

*Results: E. coli* culture grew satisfactorily and generated detectable VOCs, but all other cultures either did not achieve satisfactory growth under the initial protocol or did not produce detectable VOCs at the concentrations of stimuli used.

### B) High throughput analysis of VOC production by different human microbiome bacteria

The aims were to: (i) develop a high throughput system that maximises microbiome culture and VOC production and analysis, and (ii) examine VOCs produced by different known cancer-associated microbiome members.

The inventors set out to develop a high-throughput system as the platform for efficient testing of microbiome responses to different OSD compositions and concentrations, to inform the design of subsequent patient dosage studies, recognising that many foods and common nutritional supplements (e.g. vitamins, minerals, amino acids) often greatly exceed the RDAs for compounds potentially suitable in the OSD.

Reasons for revising the initial culture protocol: (i) insufficient microbiome growth under initial protocol in experiment (iii)A, (ii) insufficient VOC levels in response to RDA of OSD compounds in experiment (iii)A, (iii) difficulties found with the use of the OmniLog including slow growth rate of many cancer associated bacteria, and inability to maintain efficient vessel sealing during headspace sampling of highly volatile compounds, and (iv) the inventors' VOC analytical capabilities proving significantly more sensitive than Ingenza's equipment. It was therefore decided to use growth media and conditions that allowed greater culture biomass and increased concentrations of potential VOC stimuli, since *in vitro* work is not bound by patient safety constraints.

### Methods:

Growth media: The mechanisms of genetic and biochemical regulation of the gastric microbes under evaluation was considered important in deciding the composition of laboratory growth media and carbon source used to generate biomass. Glucose mediated catabolite repression can inhibit enzymes necessary to catabolise alternate carbon sources. Media rich in supplements such as amino acids or metabolic intermediates also represses bacterial biosynthesis of these compounds by enzymes that are non-essential under these conditions but whose activity may be required for VOC production. Defined minimal salts medium lacking non-essential supplementation was therefore used.

Stimuli: Concentrations of stimulant constituents were significantly increased, permitting much broader assessment of individual stimulant thresholds, temporal profiles and concerted effects of stimuli upon microbial VOC production. Microbiomes: 5 prioritised bacterial species (*Lactobacillus fermentum, Streptococcus salivarius, Streptococcus anginosus, Klebseilla pneumonia, E. coli*) were cultured with glucose or glycerol carbon sources.

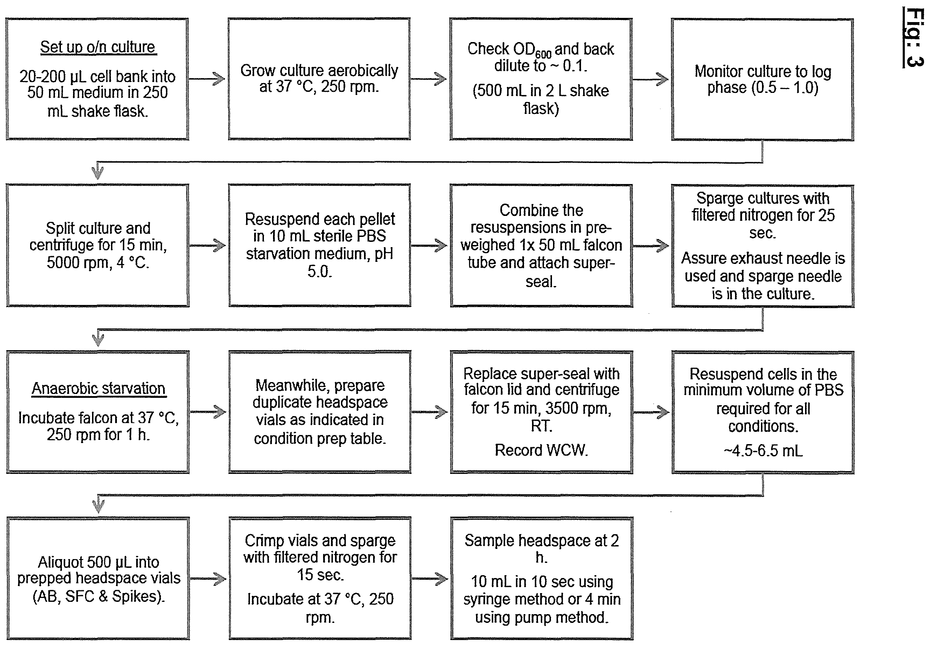

Procedure: A protocol was established for biomass generation in shake flask cultures followed by VOC stimulation in 50 ml falcon tubes. Final nitrogen sparging and high-throughput VOC sampling in headspace vials provided the required accuracy and reproducibility with no loss of throughput (see Figure 3).

VOC capture and transport: Headspace VOCs were captured on conditioned thermal desorption (TD) tubes and shipped in ice-packs to Imperial in batches of 50-100 tubes. TD tubes allow stable storage and transport of target VOCs for 72 hours at room temperature and 4 weeks at -20°C.

VOC analysis: Analysis was conducted at St Mary's VOC laboratory using standard Gas chromatography mass-spectrometry (TD/GC-MS) and Proton transfer reaction time-of-flight mass-spectrometry (TD/PTR-TOF-MS).

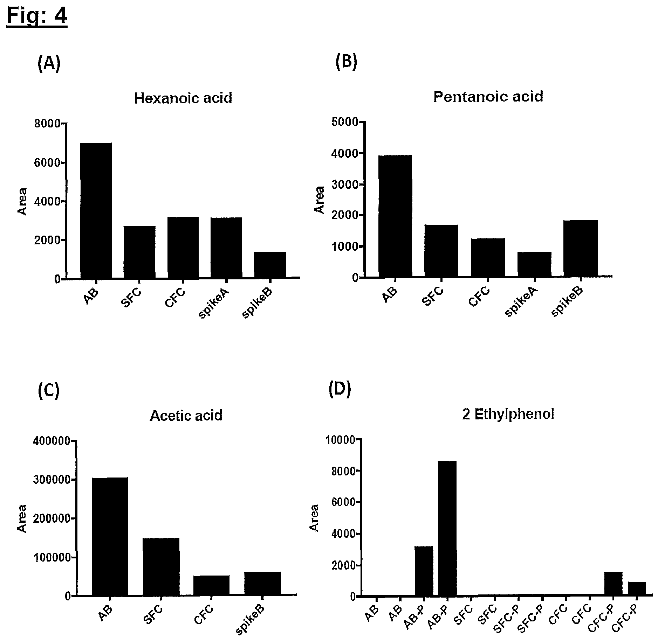

*Results:* Samples were collected for all cultures and specific VOCs found to be elevated 2-10 fold over controls in particular cultures (see Table 4 and Figure 4). The data indicated specific bacterial fatty acid, phenol and aldehyde VOCs were produced in response to the stimuli included in the culture media. Key elevated VOCs were pentanoic acid, hexanoic acid, butyric acid, acetic acid, acetaldehyde, hexanal, octanal, heptanal, phenol and ethyl phenol.

### (iv) Clinical study

### Ethical approval: REC Reference (18/LO/0078).

*Hypothesis:* Cancer cells and their associated bacteria will be able to utilise administered substrates within defined metabolic pathways responsible for the production of VOC's. By exploiting inherent metabolic pathways in this way we expect to observe a transient elevation in cancer associated VOCs.

*Methods:* Patients with cancer of the oesophageal or stomach as well as subjects with a normal upper gastrointestinal tract were recruited at the time of routine outpatient assessment. Patients were required to fast overnight prior to breath sampling. A baseline breath sample was collected at the start of the study period by asking participants to exhale directly into a double thickness Nalophan^{®} bag (Kalle UK Ltd, UK). Participants were then asked to consume the OSD. Following consumption of the OSD, participants were asked to rinse their mouth with water in order to eliminate any oral residue of the OSD. Further breath samples were then collected at 30 and 60 minutes following ingestion of the OSD. Breath samples were transferred from Nalophan^{®} bags on to thermal desorption tubes using a precision handheld pump (SKC Ltd, UK).

VOC analysis: Breath samples were analysed by PTR-TOF-MS and GC-MS techniques for target quantification of cancer biomarkers: fatty acids (acetic acid, butyric acid, pentanoic acid and hexanoic acid) phenol, ethyl phenol and aldehydes. Exhaled acetone, a marker of ketosis (a state of energy depletion) was assessed in order to verify the administration of a nutritional stimulus. Strict quality control measures were followed.

*Results:* 30 patients with gastroesophageal cancer and 30 control subjects were recruited. All participants were able to consume the OSD and there were no observed or reported adverse events. Acetone levels in both cancer and control subjects decreased following ingestion of the OSD confirming nutritional stimulation that occurred. Following ingestion of the OSD target VOCs in cancer patients (pentanoic acid, hexanoic acid, butyric acid, acetic acid, phenol, ethyl phenol) were detected at higher levels as indicated by the average fold change in VOC concentrations at 30 and 60mins. With the exception of butyric acid (30mins time point), control subjects exhibited a ≤10% variation in target VOC levels following ingestion of the OSD. Mean fold change variation in exhaled hexanoic acid, and pentanoic acid is presented in (see Table 5 and Figure 4). For cancer patients who had previously received chemoradiotherapy OSD, response for pentanoic acid appeared to be suppressed such that it was similar to control subjects.

**Table 5 - Mean fold change in select exhaled VOCs following administration of oral stimulant drink**

| | **Cancer** | | | **Controls** | | |
|---|---|---|---|---|---|---|
| | **0 mins** | **30 mins** | **60 mins** | **0 mins** | **30 mins** | **60 mins** |
| **Acetone** | 1.0 | 1.0 | 0.9 | 1.0 | 1.0 | 0.9 |
| **Acetic Acid** | 1.0 | 1.1 | 1.2 | 1.0 | 1.0 | 0.9 |
| **Butyric Acid** | 1.0 | 1.6 | 1.3 | 1.0 | 1.2 | 1.0 |
| **Pentanoic Acid** | 1.0 | 1.1 | 1.2 | 1.0 | 0.9 | 1.0 |
| **Hexanoic Acid** | 1.0 | 1.1 | 1.2 | 1.0 | 1.0 | 1.0 |
| **Phenol** | 1.0 | 1.2 | 1.3 | 1.0 | 1.0 | 0.9 |
| **Ethyl Phenol** | 1.0 | 1.2 | 1.3 | 1.0 | 0.9 | 1.0 |
| **Acetaldehyde** | 1.0 | 1.1 | 1.2 | 1.0 | 1.1 | 1.1 |

Data is derived from breath samples analysed by PTR-TOF-MS (OSD composition at the concentrations listed in Table 3: tyrosine, glutamic acid, glucose, lactose, sorbitol, glycerol, citric acid, acetic acid.

**Table 6 - Columns 2-5: Mean differences of the log-transformed data between cancer patients and controls and pooled variances at 30 and 60 minutes. Columns 6-7: Sample size calculation and power calculation in all compounds for the chosen sample size**

| Compound | Mean diff (cases - controls) time=30 min | Mean diff (cases - controls) time=60 | Pooled variance time=30 | Pooled variance time=60 | Final sample size (Power 90%) | Power with n=188 |
|---|---|---|---|---|---|---|
| Acetic Acid | 0.069 | 0.155 | 0.155 | 0.205 | 188 | 90% |
| Butyric Acid | 0.138 | 0.260 | 0.310 | 0.249 | 81 | 99.7% |
| Phenol | 0.050 | 0.232 | 0.329 | 0.420 | 171 | 92.2% |
| Pentanoic Acid | 0.139 | 0.106 | 0.164 | 0.221 | 186 | 90.2% |
| Hexanoic Acid | 0.176 | 0.203 | 0.221 | 0.168 | 90 | 99.5% |
| Ethyl Phenol | 0.219 | 0.205 | 0.113 | 0.230 | 52 | 100.0% |

The final sample size was chosen as the minimum sample size between both time points based on the expected maximum difference between cases and controls. The inventors used the formula (3.31) in Chapter 3 (Julious, SA. Sample sizes for clinical trials. 2010 - Chapman and Hall) for comparison of two means in a parallel study adjusting for the imprecision of the population variance estimation and assuming the same number of cases and controls.

VOCs belonging to fatty acids, phenols and aldehydes were produced by cancer-associated microbiomes cultured from commercial strains and cancer tissues obtained from patients with oesophago-gastric adenocarcinoma. Different microbiomes produced distinct VOC profiles. As shown in Tables 5 and 6, key elevated VOCs include pentanoic acid, hexanoic acid, butyric acid, acetic acid, butanoic acid, acetaldehyde, benzaldehyde, hexanal, octanal, heptanal, phenol, methyl phenol and ethyl phenol.

*Conclusions:* Preliminary investigations have demonstrated 'proof or principle' that exhaled biomarkers of gastroesophageal cancer could be augmented by an OSD. The findings from *in-vitro* bacterial culture experiments provide evidence that the cancer associated bacteria are, at least in part, responsible for the observed changes in target VOC. The findings have also indicated that this response may be suppressed by the prior chemoradiotherapy, which is known to modify the intestinal microbiome.

Knowledge of the VOC profile produced by different cancer-associated microbiomes can then be used to:
- Generate VOC calibration curves for GC-MS qualitative analysis,
- Test the response of microbiomes to different combinations and concentrations of OSD components in a high-throughput microbiome culture system.

### Summary

The inventors have unequivocally demonstrated an increase in the generation of VOCs in patients with oesophago-gastric cancer in comparison to non-cancer subjects in response to the oral stimulant drink (OSD). A major finding has been to obtain common stimulus-inducible VOCs in both the clinical study and in vitro microbiome culture of known cancer-associated bacteria. The inventors, therefore, have a very high confidence in the results because of the consistency of VOC identification using multiple analytical platforms (i.e. GC-MS and PTR-TOF-MS).

In addition, VOCs discovered in AMBEC are among volatile biomarkers that were found to differentiate oesophago-gastric cancer patients from control patients in the initial non-augmented breath test clinical studies (Ann Surg. 2015 Dec;262(6):981-90. JAMA Oncol. 2018 May 17). These findings provide the basis for further work with the primary objective of establishing an AMBEC protocol that achieves a higher diagnostic accuracy than the 85% shown in previous non-augmented breath analysis studies.

The novelty of the work is using the cancer-associated microbiome to elicit a diagnostic augmented VOC response. In order to realise this novelty, the inventors have achieved:
- Production of an oral drink that stimulates the cancer associated-microbiome.
- *In-vitro* demonstration of VOC production in the headspace of microbiome derived cultures in response to OSD as a means to achieve the most significant cancer dependent response *in vivo.*
- Conducting of a clinical trial that showed an increase in the generation of VOCs in patients with oesophago- gastric cancer in comparison to non-cancer subjects in response to OSD.
- Development of techniques and detailed protocols for a high throughput system for optimum culturing and testing of the cancer-associated microbiome and capturing generated VOCs, as well as VOC transport and analysis. This high throughput system is transferable to other cancers to explore microbiome augmented diagnostic responses.

### References

1. Thorn R., Reynolds D., Greenman J., Multivariate analysis of bacterial volatile compound profiles for discrimination between selected species and strains in vitro. Journal of Microbiological Methods. 2011; 84(2): 2.58-264.
2. Allardyce R., Hill A., Murdoch D., The rapid evaluation of bacterial growth and antibiotic susceptibility in blood cultures by selected ion flow tube mass spectrometry. Diagnostic Microbiology and Infectious Disease. 2006; 55(4): 255-261.
3. Julák J., Procházková-Francisci E., Stránká E., Rosová., Evalutation of exudates by solid phase microextraction-gas chromatography. Journal of Microbiological Methods. 2003; 52(1): 11.9-122.
4. Altomare DF, Di Lena M, Porcelli F, et al. Exhaled volatile organic compounds identify patients with colorectal cancer. Br J Surg 2013; 100: 144-150
5. Spanel P, Smith D. Selected ion flow tube mass spectrometry for on-line trace gas analysis in biology and medicine. Eur J Mass Spectrom 2007; 13: 77-82 12. Spanel P, Smith D, Progress in SIFT-MS: breath analysis and other applications. Mass Spectrom Rev 2011; 30: 236-267
6. Altomare DF, Di Lena M, Porcelli F, et al. Effects of curative colorectal cancer surgery on exhaled volatile organic compounds and potential implications in clinical follow-up. Ann Surg 2015; 262: 862-866.

## Claims

1. A method for diagnosing a subject suffering from oesophago-gastric junction cancer, gastric cancer, oesophageal cancer, oesophageal squamous-cell carcinoma (ESCC), or oesophageal adenocarcinoma (EAC), or a pre-disposition thereto, or for providing a prognosis of the subject's cancer, the method comprising:
(i) detecting, in a breath sample obtained from a test subject, the concentration of a signature compound resulting from the metabolism, by a cancer-associated microorganism, of at least one substrate in a composition previously administered to the subject, wherein the cancer-associated microorganism is *Streptococcus, Lactobacillus, Veillonella, Prevotella, Neisseria, Haemophilus, L. coleohominis, Lachnospiraceae, Klebsiella, Clostridiales, or Erysipelotrichales,* or any combination thereof, and the at least one substrate is selected from the group consisting of: glucose, sorbitol, lactose, tyrosine, glutamic acid, glycerol, citric acid and acetic acid, or any combination thereof; and
(ii) comparing this concentration with a reference for the concentration of the signature compound in an individual who does not suffer from cancer,
wherein the signature compound is selected from the group consisting of: acetone, acetic acid, butyric acid, pentanoic acid, hexanoic acid, phenol, ethyl phenol, acetaldehyde, or any combination thereof, and
wherein an increase or a decrease in the concentration of the signature compound compared to the reference, suggests that the subject is suffering from cancer, or has a pre-disposition thereto, or provides a negative prognosis of the subject's condition.

2. A method according to claim 1, wherein the method comprises analysing the concentration of the signature compound in the breath sample from the test subject and comparing this concentration with the reference for the concentration of the signature compound in an individual who does not suffer from cancer, optionally
wherein the cancer-associated microorganism is associated with oesophago-gastric junction cancer, gastric cancer, oesophageal cancer, oesophageal squamous-cell carcinoma (ESCC), or oesophageal adenocarcinoma (EAC).

3. A method according to any preceding claim, wherein the composition comprising the at least one substrate, which is suitable for metabolism by the cancer-associated microorganism into the signature compound, is ingestable by the subject, optionally
wherein the composition comprising the at least one substrate is: (i) in the form of a capsule that is designed to degrade at a certain position with the gastrointestinal tract, thereby offering targeted release of the at least one substrate; or (ii) is a solid, foodstuff, fluid or liquid, which was swallowed.

4. A method according to any preceding claim, wherein the cancer-associated microorganism is:
(i) *S. pyogenes, Klebsiella pneumoniae, Lactobacillus acidophilus,* or any combination thereof, and/or
(ii) *Streptococcus salivarius, Streptococcus anginosus, S. pyogenes, Lactobacillus fermentum, Clostridium bifermentans, Clostridium perfringens, Clostridium septicum, Clostridium sporogenes, Clostridium tertium, Lactobacillus acidophilus, S. pneumoniae, N. meningitides, Clostridium difficile, Clostridium ramosum, Klebsiella pneumoniae,* or any combination thereof.

5. A method according to any preceding claim, wherein the method is used to provide a diagnosis, indicate a pre-disposition thereto, or provide a prognosis of, oesophageal squamous-cell carcinoma, wherein the composition comprises acetic acid, which is metabolised to a signature compound butyric acid, optionally which is analysed to indicate the presence of *Clostridium spp.*

6. Use of a composition in the *in vitro* diagnosis or prognosis of oesophago-gastric junction cancer, gastric cancer, oesophageal cancer, oesophageal squamous-cell carcinoma (ESCC), or oesophageal adenocarcinoma (EAC), according to the method of any one of claims 1-5, wherein the composition consists of one or more substrate selected from the group consisting of: glucose, sorbitol, lactose, tyrosine, glutamic acid, glycerol, citric acid and acetic acid, or any combination thereof, and wherein the at least one substrate is metabolised by a cancer-associated microorganism into a signature compound selected from the group consisting of: acetone, acetic acid, butyric acid, pentanoic acid, hexanoic acid, phenol, ethyl phenol, acetaldehyde, or any combination thereof.

7. Use of the composition according to claim 6, wherein the composition comprises:
(i) glucose at a concentration of between about 7000mg/100mL and 20000mg/100mL, or between about 9000mg/100mL and 17000mg/100mL, or between about 11000mg/100mL and 15000mg/100mL;
(ii) lactose at a concentration of between about 7000mg/100mL and 20000mg/100mL, or between about 9000mg/100mL and 17000mg/100mL, or between about 11000mg/100mL and 15000mg/100mL;
(iii) sorbitol at a concentration of between about 1000mg/100mL and 6000mg/100mL, or between about 2000mg/100mL and 5000mg/100mL, or between about 3000mg/100mL and 4000mg/100mL;
(iv) tyrosine at a concentration of between about 25mg/100mL and 500mg/100mL, or between about 50mg/100mL and 400mg/100mL, or between about 100mg/100mL and 300mg/100mL;
(v) glutamic acid at a concentration of between about 500mg/100mL and 5000mg/100mL, or between about 1000mg/100mL and 3500mg/100mL, or between about 1500mg/100mL and 2500mg/100mL;
(vi) glycerol at a concentration of between about 10000mg/100mL and 30000mg/100mL, or between about 14000mg/100mL and 25000mg/100mL, or between about 17000mg/100mL and 22000mg/100mL;
(vi) citric acid at a concentration of between about 500mg/100mL and 3000mg/100mL, or between about 1000mg/100mL and 2000mg/100mL, or between about 1200mg/100mL and 1700mg/100mL; and/or
(vii) acetic acid at a concentration of between about 200mg/100mL and 1500mg/100mL, or between about 400mg/100mL and 1000mg/100mL, or between about 600mg/100mL and 800mg/100mL.

8. Use of a kit in the *in vitro* diagnosis of oesophago-gastric junction cancer, gastric cancer, oesophageal cancer, oesophageal squamous-cell carcinoma (ESCC), or oesophageal adenocarcinoma (EAC), or a pre-disposition thereto, or in the prognosis of oesophago-gastric junction cancer, gastric cancer, oesophageal cancer, oesophageal squamous-cell carcinoma (ESCC), or oesophageal adenocarcinoma (EAC), wherein the kit is for performing the method according to any one of claims 1-5, the kit comprising:-
(a) a composition consisting of one or more substrate selected from the group consisting of: glucose, sorbitol, lactose, tyrosine, glutamic acid, glycerol, citric acid and acetic acid, or any combination thereof;
(b) means for determining the concentration of a signature compound resulting from the metabolism, by a cancer-associated microorganism, of the at least one substrate, in a breath sample from a test subject, wherein the signature compound is selected from the group consisting of: acetone, acetic acid, butyric acid, pentanoic acid, hexanoic acid, phenol, ethyl phenol, acetaldehyde, or any combination thereof; and
(c) a reference for the concentration of the signature compound in a breath sample from an individual who does not suffer from cancer,
wherein the kit is used to identify an increase or a decrease in the concentration of the signature compound in the breath sample from the test subject, compared to the reference, thereby suggesting that the subject suffers from cancer, or has a pre-disposition thereto, or provides a negative prognosis of the subject's condition.

## Patentansprüche

1. Verfahren zum Diagnostizieren eines Subjekts, das an Adenokarzinom des gastroösophagealen Übergangs, Magenkarzinom, Ösophaguskarzinom, Ösophagus-Plattenepithelkarzinom (ESCC) oder Ösophagus-Adenokarzinom (EAC) leidet, oder einer Prädisposition dafür, oder zum Liefern einer Prognose des Krebses des Subjekts, wobei das Verfahren umfasst:
(i) Detektieren der Konzentration einer Signaturverbindung, die aus dem Metabolismus mindestens eines Substrats in einer zuvor dem Subjekt verabreichten Zusammensetzung durch einen krebsassoziierten Mikroorganismus resultiert, in einer von einem Testsubjekt erhaltenen Atemprobe, wobei der krebsassoziierte Mikroorganismus *Streptococcus, Lactobacillus, Veillonella, Prevotella, Neisseria, Haemophilus, L. coleohominis, Lachnospiraceae, Klebsiella, Clostridiales oder Erysipelotrichales* oder eine Kombination davon ist und das mindestens eine Substrat ausgewählt ist aus der Gruppe bestehend aus: Glucose, Sorbitol, Lactose, Tyrosin, Glutaminsäure, Glycerin, Zitronensäure und Essigsäure oder einer Kombination davon; und
(ii) Vergleichen dieser Konzentration mit einer Referenz für die Konzentration der Signaturverbindung in einem Individuum, das nicht an Krebs leidet, wobei die Signaturverbindung ausgewählt ist aus der Gruppe bestehend aus: Aceton, Essigsäure, Buttersäure, Pentansäure, Hexansäure, Phenol, Ethylphenol, Acetaldehyd oder einer Kombination davon, und
wobei eine Zunahme oder Abnahme der Konzentration der Signaturverbindung verglichen mit der Referenz darauf hindeutet, dass das Subjekt an Krebs leidet oder eine Prädisposition dafür aufweist, oder eine negative Prognose des Zustands des Subjekts liefert.

2. Verfahren gemäß Anspruch 1, wobei das Verfahren das Analysieren der Konzentration der Signaturverbindung in der Atemprobe von dem Testsubjekt und das Vergleichen dieser Konzentration mit der Referenz für die Konzentration der Signaturverbindung in einem Individuum, das nicht an Krebs leidet, umfasst, wobei der krebsassoziierte Mikroorganismus optional mit Adenokarzinom des gastroösophagealen Übergangs, Magenkarzinom, Ösophaguskarzinom, Ösophagus-Plattenepithelkarzinom (ESCC) oder Ösophagus-Adenokarzinom (EAC) assoziiert ist.

3. Verfahren gemäß einem vorhergehenden Anspruch, wobei die Zusammensetzung, umfassend das mindestens eine Substrat, das für den Metabolismus durch den krebsassoziierten Mikroorganismus in die Signaturverbindung geeignet ist, von dem Subjekt aufgenommen werden kann, optional
wobei die Zusammensetzung, die das mindestens eine Substrat umfasst, wie folgt ist: (i) in der Form einer Kapsel, die ausgelegt ist, um an einer bestimmten Position mit dem Gastrointestinaltrakt abgebaut zu werden, wodurch eine gezielte Freisetzung des mindestens einen Substrats angeboten wird; oder (ii) ein Feststoff, ein Lebensmittel, ein Fluid oder eine Flüssigkeit ist, der/die geschluckt wird.

4. Verfahren gemäß einem vorhergehenden Anspruch, wobei der krebsassoziierte Mikroorganismus Folgendes ist:
(i) *S. pyogenes, Klebsiella pneumoniae, Lactobacillus acidophilus,* oder eine Kombination davon, und/oder
(ii) *Streptococcus salivarius, Streptococcus anginosus, S. pyogenes, Lactobacillus fermentum, Clostridium bifermentans, Clostridium perfringens, Clostridium septicum, Clostridium sporogenes, Clostridium tertium, Lactobacillus acidophilus, S. pneumoniae, N. meningitides, Clostridium difficile, Clostridium ramosum, Klebsiella pneumoniae,* oder eine Kombination davon.

5. Verfahren gemäß einem vorhergehenden Anspruch, wobei das Verfahren verwendet wird, um eine Diagnose zu liefern, eine Prädisposition dafür anzuzeigen oder eine Prognose eines Plattenepithelkarzinoms der Speiseröhre zu liefern, wobei die Zusammensetzung Essigsäure umfasst, die zu einer Signaturverbindung Buttersäure metabolisiert wird, die optional analysiert wird, um das Vorhandensein von *Clostridium spp.* anzuzeigen.

6. Verwendung einer Zusammensetzung bei der *In-vitro-Diagnose* oder -Prognose von Adenokarzinom des gastroösophagealen Übergangs, Magenkarzinom, Ösophaguskarzinom, Ösophagus-Plattenepithelkarzinom (ESCC) oder Ösophagus-Adenokarzinom (EAC), gemäß dem Verfahren nach einem der Ansprüche 1-5, wobei die Zusammensetzung aus einem oder mehreren Substraten besteht, ausgewählt aus der Gruppe bestehend aus: Glucose, Sorbitol, Lactose, Tyrosin, Glutaminsäure, Glycerin, Zitronensäure und Essigsäure oder einer Kombination davon, und wobei das mindestens eine Substrat durch einen krebsassoziierten Mikroorganismus zu einer Signaturverbindung metabolisiert wird, die ausgewählt ist aus der Gruppe bestehend aus: Aceton, Essigsäure, Buttersäure, Pentansäure, Hexansäure, Phenol, Ethylphenol, Acetaldehyd oder einer Kombination davon.

7. Verwendung der Zusammensetzung gemäß Anspruch 6, wobei die Zusammensetzung umfasst:
(i) Glucose in einer Konzentration zwischen etwa 7000 mg/100 ml und 20000 mg/100 ml oder zwischen etwa 9000 mg/100 ml und 17000 mg/100 ml oder zwischen etwa 11000 mg/100 ml und 15000 mg/100 ml;
(ii) Lactose in einer Konzentration zwischen etwa 7000 mg/100 ml und 20000 mg/100 ml oder zwischen etwa 9000 mg/100 ml und 17000 mg/100 ml oder zwischen etwa 11000 mg/100 ml und 15000 mg/100 ml;
(iii) Sorbitol in einer Konzentration zwischen etwa 1000 mg/100 ml und 6000 mg/100 ml oder zwischen etwa 2000 mg/100 ml und 5000 mg/100 ml oder zwischen etwa 3000 mg/100 ml und 4000 mg/100 ml;
(iv) Tyrosin in einer Konzentration zwischen etwa 25 mg/100 ml und 500 mg/100 ml oder zwischen etwa 50 mg/100 ml und 400 mg/100 ml oder zwischen etwa 100 mg/100 ml und 300 mg/100 ml;
(v) Glutaminsäure in einer Konzentration zwischen etwa 500 mg/100 ml und 5000 mg/100 ml oder zwischen etwa 1000 mg/100 ml und 3500 mg/100 ml oder zwischen etwa 1500 mg/100 ml und 2500 mg/100 ml;
(vi) Glycerin in einer Konzentration zwischen etwa 10000 mg/100 ml und 30000 mg/100 ml oder zwischen etwa 14000 mg/100 ml und 25000 mg/100 ml oder zwischen etwa 17000 mg/100 ml und 22000 mg/100 ml;
(vi) Zitronensäure in einer Konzentration zwischen etwa 500 mg/100 ml und 3000 mg/100 ml oder zwischen etwa 1000 mg/100 ml und 2000 mg/100 ml oder zwischen etwa 1200 mg/100 ml und 1700 mg/100 ml; und/oder
(vii) Essigsäure in einer Konzentration zwischen etwa 200 mg/100 ml und 1500 mg/100 ml oder zwischen etwa 400 mg/100 ml und 1000 mg/100 ml oder zwischen etwa 600 mg/100 ml und 800 mg/100 ml.

8. Verwendung eines Kits bei der *In-vitro-Diagnose* von Adenokarzinom des gastroösophagealen Übergangs, Magenkarzinom, Ösophaguskarzinom, Ösophagus-Plattenepithelkarzinom (ESCC) oder Ösophagus-Adenokarzinom (EAC) oder einer Prädisposition dafür oder bei der Prognose von Adenokarzinom des gastroösophagealen Übergangs, Magenkarzinom, Ösophaguskarzinom, Ösophagus-Plattenepithelkarzinom (ESCC) oder Ösophagus-Adenokarzinom (EAC), wobei das Kit zum Durchführen des Verfahrens gemäß einem der Ansprüche 1-5 dient, wobei das Kit Folgendes umfasst:-
(a) eine Zusammensetzung, bestehend aus einem oder mehreren Substraten, die ausgewählt sind aus der Gruppe bestehend aus: Glucose, Sorbit, Lactose, Tyrosin, Glutaminsäure, Glycerin, Zitronensäure und Essigsäure oder einer Kombination davon;
(b) Mittel zum Bestimmen der Konzentration einer Signaturverbindung, die aus dem Metabolismus des mindestens einen Substrats durch einen krebsassoziierten Mikroorganismus resultiert, in einer Atemprobe von einem Testsubjekt, wobei die Signaturverbindung ausgewählt ist aus der Gruppe bestehend aus: Aceton, Essigsäure, Buttersäure, Pentansäure, Hexansäure, Phenol, Ethylphenol, Acetaldehyd oder einer Kombination davon; und
(c) eine Referenz für die Konzentration der Signaturverbindung in einer Atemprobe von einem Individuum, das nicht an Krebs leidet,
wobei das Kit verwendet wird, um eine Zunahme oder Abnahme der Konzentration der Signaturverbindung in der Atemprobe von dem Testsubjekt verglichen mit der Referenz zu identifizieren, was darauf hindeutet, dass das Subjekt an Krebs leidet oder eine Prädisposition dafür aufweist, oder eine negative Prognose des Zustands des Subjekts liefert.

## Revendications

1. Méthode permettant de diagnostiquer un sujet atteint d'un cancer de la jonction œsophago-gastrique, d'un cancer de l'estomac, d'un cancer de l'œsophage, d'un carcinome épidermoïde de l'œsophage (CEO) ou d'un adénocarcinome de l'œsophage (ACO), ou d'une prédisposition à ceux-ci, ou permettant d'établir un pronostic du cancer du sujet, la méthode comprenant :
(i) la détection, dans un échantillon d'haleine prélevé chez un sujet testé, de la concentration d'un composé signature résultant du métabolisme, par un micro-organisme associé à un cancer, d'au moins un substrat dans une composition précédemment administrée au sujet, ledit micro-organisme associé à un cancer étant *Streptococcus, Lactobacillus, Veillonella, Prevotella, Neisseria, Haemophilus, L. coleohominis, Lachnospiraceae, Klebsiella, Clostridiales ou Erysipelotrichales,* ou toute combinaison de ceux-ci, et ledit au moins un substrat étant choisi dans le groupe constitué par : le glucose, le sorbitol, le lactose, la tyrosine, l'acide glutamique, le glycérol, l'acide citrique et l'acide acétique, ou toute combinaison de ceux-ci ; et
(ii) la comparaison de cette concentration à une référence pour la concentration du composé signature chez un individu qui n'est pas atteint d'un cancer,
ledit composé signature étant choisi dans le groupe constitué par : l'acétone, l'acide acétique, l'acide butyrique, l'acide pentanoïque, l'acide hexanoïque, le phénol, l'éthylphénol, l'acétaldéhyde, ou toute combinaison de ceux-ci, et
une augmentation ou une diminution de la concentration du composé signature par rapport à la référence, suggérant que le sujet est atteint d'un cancer, ou présente une prédisposition à celui-ci, ou établissant un pronostic négatif de l'état du sujet.

2. Méthode selon la revendication 1, ladite méthode comprenant l'analyse de la concentration du composé signature dans l'échantillon d'haleine du sujet testé et la comparaison de cette concentration à la référence pour la concentration du composé signature chez un individu qui n'est pas atteint d'un cancer, éventuellement
ledit micro-organisme associé à un cancer étant associé à un cancer de la jonction œsophago-gastrique, à un cancer de l'estomac, à un cancer de l'esophage, à un carcinome épidermoïde de l'œsophage (CEO) ou à un adénocarcinome de l'esophage (ACO).

3. Méthode selon l'une quelconque des revendications précédentes, ladite composition comprenant ledit au moins un substrat, qui est approprié pour le métabolisme par le micro-organisme associé à un cancer en composé de signature, étant ingérable par le sujet, éventuellement
ladite composition comprenant ledit au moins un substrat étant : (i) sous la forme d'une capsule qui est conçue pour se dégrader dans une certaine position avec le tractus gastro-intestinal, offrant ainsi une libération ciblée dudit au moins un substrat ; ou (ii) étant un solide, un aliment, un fluide ou un liquide, qui a été avalé.

4. Méthode selon l'une quelconque des revendications précédentes, ledit micro-organisme associé à un cancer étant :
(i) *S. pyogenes, Klebsiella pneumoniae, Laetobaeillus acidophilus,* ou toute combinaison de ceux-ci, et/ou
(ii) *Streptococcus salivarius, Streptococcus anginosus, S. pyogenes, Lactobacillus fermentum, Clostridium bifermentans, Clostridium perfringens, Clostridium septicum, Clostridium sporogenes, Clostridium tertium, Lactobacillus acidophilus, S. pneumoniae, N. meningitides, Clostridium difficile, Clostridium ramosum, Klebsiella pneumoniae,* ou toute combinaison de ceux-ci.

5. Méthode selon l'une quelconque des revendications précédentes, ladite méthode étant utilisée pour établir un diagnostic, indiquer une prédisposition à celui-ci, ou établir un pronostic de carcinome épidermoïde œsophagien, ladite composition comprenant de l'acide acétique, qui est métabolisé en un composé signature acide butyrique, qui est éventuellement analysé pour indiquer la présence de *Clostridium spp.*

6. Utilisation d'une composition dans le diagnostic ou le pronostic *in vitro* du cancer de la jonction œsophago-gastrique, du cancer de l'estomac, du cancer de l'œsophage, d'un carcinome épidennoïde de l'œsophage (CEO) ou d'un adénocarcinome de l'œsophage (ACO), conformément à la méthode selon l'une quelconque des revendications 1 à 5, ladite composition se composant d'un ou plusieurs substrats choisis dans le groupe constitué par : le glucose, le sorbitol, le lactose, la tyrosine, l'acide glutamique, le glycérol, l'acide citrique et l'acide acétique, ou toute combinaison de ceux-ci, et ledit au moins un substrat étant métabolisé par un micro-organisme associé à un cancer en un composé signature choisi dans le groupe constitué par : l'acétone, l'acide acétique, l'acide butyrique, l'acide pentanoïque, l'acide hexanoïque, le phénol, l'éthylphénol, l'acétaldéhyde, ou toute combinaison de ceux-ci.

7. Utilisation de la composition selon la revendication 6, ladite composition comprenant :
(i) du glucose à une concentration comprise entre environ 7000 mg/100 ml et 20000 mg/100 ml, ou entre environ 9000 mg/100 ml et 17000 mg/100 ml, ou entre environ 11000 mg/100 ml et 15000 mg/100 ml ;
(ii) du lactose à une concentration comprise entre environ 7000 mg/100 ml et 20000 mg/100 ml, ou entre environ 9000 mg/100 ml et 17000 mg/100 ml, ou entre environ 11000 mg/100 ml et 15000 mg/100 ml ;
(iii) du sorbitol à une concentration comprise entre environ 1000 mg/100 ml et 6000 mg/100 ml, ou entre environ 2000 mg/100 ml et 5000 mg/100 ml, ou entre environ 3000 mg/100 ml et 4000 mg/100 ml ;
(iv) de la tyrosine à une concentration comprise entre environ 25 mg/100 ml et 500 mg/100 ml, ou entre environ 50 mg/100 ml et 400 mg/100 ml, ou entre environ 100 mg/100 ml et 300 mg/100 ml ;
(v) de l'acide glutamique à une concentration comprise entre environ 500 mg/100 ml et 5000 mg/100 ml, ou entre environ 1000 mg/100 ml et 3500 mg/100 ml, ou entre environ 1500 mg/100 ml et 2500 mg/100 ml ;
(vi) du glycérol à une concentration comprise entre environ 10000 mg/100 ml et 30000 mg/100 ml, ou entre environ 14000 mg/100 ml et 25000 mg/100 ml, ou entre environ 17000 mg/100 ml et 22000 mg/100 ml ;
(vi) de l'acide citrique à une concentration comprise entre environ 500 mg/100 ml et 3000 mg/100 ml, ou entre environ 1000 mg/100 ml et 2000 mg/100 ml, ou entre environ 1200 mg/100 ml et 1700 mg/100 ml ; et/ou
(vii) de l'acide acétique à une concentration comprise entre environ 200 mg/100 ml et 1500 mg/100 ml, ou entre environ 400 mg/100 ml et 1000 mg/100 ml, ou entre environ 600 mg/100 ml et 800 mg/100 ml.

8. Utilisation d'un kit dans le diagnostic *in vitro* du cancer de la jonction œsophago-gastrique, du cancer de l'estomac, du cancer de l'œsophage, d'un carcinome épidennoïde de l'œsophage (CEO) ou d'un adénocarcinome de l'œsophage (ACO), ou d'une prédisposition à ceux-ci, ou dans le pronostic du cancer de la jonction œsophago-gastrique, du cancer de l'estomac, du cancer de l'œsophage, d'un carcinome épidermoïde de l'œsophage (CEO) ou d'un adénocarcinome de l'œsophage (ACO), ledit kit étant destiné à la réalisation de la méthode selon l'une quelconque des revendications 1 à 5, ledit kit comprenant :
(a) une composition constituée d'un ou plusieurs substrats choisis dans le groupe constitué par : le glucose, le sorbitol, le lactose, la tyrosine, l'acide glutamique, le glycérol, l'acide citrique et l'acide acétique, ou toute combinaison de ceux-ci ;
(b) un moyen pour déterminer la concentration d'un composé signature résultant du métabolisme, par un micro-organisme associé à un cancer, dudit au moins un substrat, dans un échantillon d'haleine d'un sujet testé, ledit composé signature étant choisi dans le groupe constitué par : l'acétone, l'acide acétique, l'acide butyrique, l'acide pentanoïque, l'acide hexanoïque, le phénol, l'éthylphénol, l'acétaldéhyde, ou toute combinaison de ceux-ci ; et
(c) une référence pour la concentration du composé signature dans un échantillon d'haleine d'un individu qui n'est pas atteint d'un cancer,
ledit kit étant utilisé pour identifier une augmentation ou une diminution de la concentration du composé signature dans l'échantillon d'haleine du sujet testé, par rapport à la référence, suggérant ainsi que le sujet est atteint d'un cancer, ou présente une prédisposition à celui-ci, ou établissant un pronostic négatif de l'état du sujet.
